# EUROPEAN PATENT APPLICATION

(11) **EP 4 368 238 A1**
(43) Date of publication of application: **15.05.2024**
(21) Application number: 22206219.2
(22) Date of filing: 08.11.2022
(51) Int. Cl.: A61N 1/05, A61N 1/36

(54) **A COMPUTER-IMPLEMENTED METHOD OF MAPPING ELECTRODES OF A BRAIN PROSTHESIS**

(71) Applicant: Nederlands Herseninstituut, 1105 BA Amsterdam (NL)
(72) Inventor: ROELFSEMA, Pieter R., Amsterdam (NL); CHEN, Xing, Amsterdam (NL); LI, Bingshuo, Amsterdam (NL); LOZANO ORTEGA, Antonio Manuel, Amsterdam (NL); WANG, Feng, Amsterdam (NL); LA GROUW, Mike, Amsterdam (NL)
(74) Representative: Algemeen Octrooi- en Merkenbureau B.V.

(57) **Abstract**

An aspect of the present disclosure relates to a computer-implemented method for mapping electrodes of a brain prosthesis for substituting a defective functional modality of a mammal with one or more electrode arrays for stimulation of and recording from a region of the brain of said mammal corresponding to a neural modality for which said functional modality is to be substituted, wherein automated mapping is combined with manual mapping.

## Description

### Technical Field

The present disclosure relates to the field of biomedical engineering and, in particular, to a neuroprosthetics system, and more in particular a method to fit an algorithm for a brain prosthesis, determining the relative positions of electrodes of the brain prosthesis, to substitute or induce a missing or impaired functionality (e.g. sensory or motor) of a mammal, e.g. by electrical stimulation of intracortical electrodes, for example to restore functional vision of a human being suffering from partial or total blindness.

### Background

Neuroprosthetics or neural prosthetics, is a technical discipline related to neuroscience and biomedical engineering concerned with the development of neural prostheses. A neural sensory prosthesis, in its most general form, is a device or system designed to substitute or induce, by neural stimulation in a respective region of the cerebral cortex of a mammal, a sensory functionality, such as visual, auditory, tactile, olfactory, or a motor functionality, that might have been damaged as a result of an injury or a disease.

In the field of sensory restoration of visual functionality, for example, damages in the visual system can be generally classified into two groups, i.e. a first group with damage in the visual processing pathway up to and including the ganglion cell layer in the retina, and a second group with damage after this processing stage impairing information flow between the retina and the visual cortex.

For the first group of patients, significant progress has been made towards solutions in the form of retinal prostheses for implantation in the retina, providing low vision to mammals, in which the retinal ganglion cells that connect the eye to the brain have been spared. Retinal implants cannot be used for people with extensive damage to the ganglion cells and/or optic nerve.

For the large group of patients whose sight cannot be restored in the retina, i.e. the above-mentioned second group, prostheses comprised of surface stimulation electrodes for application over the exterior surface of the visual cortex have been developed, as well as prostheses comprising intracortical electrodes in the visual cortex, for activation of a sparse subset of phosphene locations.

European patent EP3632503B1, discloses a neuroprosthetics system for substituting or inducing a missing or impaired sensory functionality of a mammal, by electrical stimulation of intracortical electrodes, for example to restore functional vision of a human being suffering from partial or total blindness.

US patent application 2010/0094382 discloses a visual prosthesis system operative to deliver electrical signals to the lateral geniculate nucleus of the thalamus of a mammal, which is the part of the thalamus that relays visual information from the eye to the cerebral cortex. The system comprises an array of rigid electrodes, a visual information translator operatively connected to the electrodes, and a visual sensor operatively connected to the array of electrodes. The visual translator is arranged for translating the visual information provided by the visual sensor for stimulating the lateral geniculate nucleus with an electrical signal through the electrodes in a manner to stimulate a subject's brain activity to recognize visual information.

Brain prostheses or neuroprosthetics rely on these electrode arrays for sensory restoration to help a mammal to restore the ability to perceive certain defective sensory modalities by using sensory information from a functioning modality or a prosthesis replacing such functionality. These sensory modalities include: light, sound, temperature, taste, pressure, and smell.

A typical brain or neuroprosthetic system is a visual prosthesis system, more precisely a visual cortical or thalamic prosthesis, in which a rudimentary form of vision is provided for the blind.

As blindness affects over 40 million individuals worldwide, it results in substantial decreases in mean employment rate, income, and quality of life. Technological advances in brain-machine interfaces over the past decades have demonstrated that the delivery of electrical currents to the visual cortex or to the lateral geniculate nucleus of the thalamus via implanted electrodes, e.g. in the electrode arrays mentioned above, results in the generation of artificial visual precepts known as 'phosphenes.' By attaching a camera to a pair of glasses, sending the video feed to a pocket processor for image processing, sending instructions to an implant in the visual cortex, and stimulating the tissue via a high-channel-count interface, the user would experience a rudimentary form of functional vision.

As it may be unclear what the location of the electrodes in the brain structure is, a mapping of the electrodes of the prosthesis is required before it can be used. The goal is to determine the relation between the channel or electrode identity on the prosthesis, and the location of the electrodes in the brain structure/functional map. From the location of electrodes within the map, the location of phosphenes can deduced.

Typical mapping routines for a (e.g. visual cortical) prosthesis may require driving or stimulating each and every electrode and obtaining user input as an indication of where the user perceived the stimulation (e.g. phosphene in case of a visual cortical prosthesis).

Such a manual phosphene mapping method requires a calibration environment, and typically, trained staff to aid with the mapping routine. Recent developments to increase the density of the electrodes on the electrode arrays, as well as the miniaturization of these arrays, enable high-resolution visual cortical prostheses which improve the artificial function vision and make it less rudimentary.

Such increase in electrodes however also increases the complexity and length of the mapping routine, e.g. the mapping of the phosphenes. Performing a manual phosphene mapping routine is considered labour-intensive and time-consuming for both the trained staff as for the user.

As such, there is a need for an improved mapping method for the relation between perceived sensory modalities coordinates, e.g. phosphene locations, and electrodes in the brain of a mammal for use in a brain or neuro prosthesis such as a visual brain prosthesis.

### Summary

The above mentioned and other objectives are achieved, in a first aspect of the present disclosure, by a computer-implemented method for mapping electrodes of a brain prosthesis for substituting a defective functional modality of a mammal with one or more electrode arrays for stimulation of and recording from a region of the brain of said mammal corresponding to a neural modality for which said functional modality is to be substituted, said method being performed by a computing unit and comprising the steps of:
- obtaining a neural dataset, wherein said neural dataset comprises data simultaneously sensed from each electrode of said one or more electrode arrays;
- defining a relative location map from said neural dataset;
- obtaining input data, wherein said input data is obtained from said mammal as feedback on driving a selected subset of electrodes for stimulation of said region of said brain, and wherein said input data comprises a set of location data in absolute target space, and each of said location data corresponds to an electrode of said subset;
- corresponding each of said location data with a data point in said relative location map, corresponding to said electrode of said subset;
- calculating location data for remaining electrodes, not comprised in said subset;
- defining an absolute location map comprising location data of each electrode of said one or more electrode arrays, which map defines sensory perception locations in absolute visual space, and wherein said location data comprises both said calculated location data and location data obtained from said input data of said mammal;
- generating a sensory perception location mapping for electrically driving or recording from said electrodes of said one or more electrode arrays for subsets of said region of the brain in accordance with said defined absolute location map, for said functional modality.

The present disclosure is based on the insight that functional substitution of an impaired functional modality necessitates electrical stimulation of numerous locations in a respective region of the cerebral cortex providing functional coverage of the sensory modality.

Some regions of the cerebral cortex have complex shapes. The visual region V1, for example, has a folded structure. The present disclosure may rely on over-dimensioning of the electrodes to provide sufficient coverage of these regions. Although some electrodes may actually fall outside of this region of interest, e.g. visual region V1, still a large number of electrodes cover this region in such a sufficiently widespread manner that the sensory modality can be substituted in a functional manner.

The term functional manner refers to at least such restoration of the sensory modality to a level sufficient for the patient to improve their independence and quality of life. This would not necessarily require full restoration of the sensory modality but at least such restoration beyond basic functioning in which only proof-of-concept is achieved.

This is achieved by providing plural three-dimensional arrays of electrodes, which preferably are flexible electrode shafts attached to a rigid electrode support structure or temporarily stiffened to allow insertion into the tissue, which are arranged for insertion in a simultaneous manner (i.e. in one single step in which all electrode shafts of one array are displaced together instead of one-by-one), wherein all electrode shafts of an array are inserted into a target region of the cerebral cortex of the mammal, and for retracting the support structure (if present) after implantation of the flexible electrode shafts.

The over-dimensioning provides a dense set of very thin, preferably flexible, electrode shafts for uniform stimulation of locations in the cortex. Thereby cortical areas that are otherwise difficult to access, can be stimulated, and recorded from, such as in a sulcus of the cerebral cortex. In this way, these areas have such a widespread distribution of electrodes in said area of the cerebral cortex of the patient or mammal that functional coverage of the sensory modality is achieved.

High-channel-count stimulation of the cortex provides a functional substitution of the missing neural modality, that is to restore for example vision in a meaningful manner providing functional coverage.

The term three-dimensional array refers to the fact that the electrical contacts for stimulation and/or reception of stimuli occupy a three-dimensional volume or region.

Implanting several arrays, each having a dense set of electrodes provides potentially a high resolution of functional sensory perceptions, e.g. phosphenes in the case of a visual prosthesis. Increasing the number of electrodes, potentially, increases the resolution of the sensory perception. Such dense electrode arrays, especially when several of such arrays are implanted, thus potentially provide a high resolution of sensory perception sufficient for providing at least basic substitution of the missing functional modality for the mammal.

Throughout the description, in several aspects of the present disclosure as well as in their corresponding embodiments, a visual prosthesis is described, and in particular an intracortical visual prosthesis, as an example in which the present invention may be embodied. It is emphasized that this is thus only an example, and that other types of defective sensory and/or functional modalities may also be replaced by calibration and subsequent stimulation of such electrodes arrays in accordance with the present invention, when the electrode arrays are implanted in other regions of the brain. In the example of the visual prosthesis, the high number of electrodes substitute the visual modality by generating numerous phosphenes by stimulating and recording from the visual cortex through the electrodes.

Implanting such electrode arrays thus, in case of a visual prosthesis, potentially provides the possibility to generate phosphenes for each electrode of the arrays and thus at a plurality of particular locations in the visual field. The visual field in the present disclosure is also referred to as the absolute target space, which corresponds to a map with retinal coordinates or visual field coordinates in which the locations are defined by a degree of visual angle (dva). Increasing the number of electrodes, as mentioned, increases the number of phosphenes, although potentially, as not each and every implanted electrode may generate a phosphene, due to several technical and neurologic reasons, and the locations of phosphenes generated via multiple electrodes may be overlapping or identical.

Moreover, prior to use of any intracortical or subcortical prosthesis, such as the described intracortical visual prosthesis, the electrodes need to be calibrated to determine, for each and every electrode, its reference in the absolute world. In case of the visual prosthesis, before it can be taken into use by the mammal for stimulating and generating phosphenes, the prosthesis needs to be calibrated to determine, for each electrode, the location in the visual field at which each phosphene is expected to appear. With the high and increasing number of electrodes, mapping becomes more and more complex and requires lengthy mapping routines.

The present disclosure is based on the insight that such mapping may be performed in a hybrid manner in which manual mapping techniques are combined in an inventive manner with an automated procedure of mapping, which obviates the drawbacks of conventional manual mapping routines, making the mapping scalable for larger numbers of electrodes. The proposed method is also more robust than conventional manual mapping routines, even under the occurrence of spontaneous phosphenes and abnormal eye movements.

What is proposed is to obtain neural data, e.g. by recording. The neural data is data that is being sensed from the electrodes, in a simultaneous manner. This step involves recording a large number of data elements from the implanted electrodes, for example when the mammal is asleep, although the mammal may also be awake. During this recording step, there may be no stimulation of the electrodes, hence the electrodes are operated as sensors, and not driven to generate brain stimulation signals. In case of a visual prosthesis, no visual input via the eye and no phosphene generation are required, in the case where the patient is completely blind. It is emphasized that the presented method is also applicable for patients who are not completely blind, as there may be residual visual input.

The thus obtained data, i.e. referred to as neural data comprising the plurality of neural data elements, is used to calculate or convert the data into maps of relative phosphene locations. These are the locations of phosphenes of all the electrodes, wherein the locations are relative to each other. With this step the thus created data is converted into a map representing the relative positions of the electrodes in the implanted brain region(s).

Once the relative locations are mapped, these are thus transformed and may be represented as a two-dimensional map of the brain data elements. As the two-dimensional map contains information on the positions of each electrode relative to each other, the map may also be represented as a three-dimensional map or a curved-plane in which the position or the relative position of the mapped channels within the 2D or 3D or curved-plane space represent the relation, correlation or level of similarity.

Once the step of automated, high-volume, mapping is completed, and relative information is represented in a map function, a relation with absolute space may be made in the following steps. This is achieved by carrying out a limited, i.e. low volume, of manual mapping actions in which a subset of the electrodes are driven, and thereby the corresponding implanted cortical region stimulated. The mammal, e.g. person undergoing the calibration routine, is requested to provide feedback from the stimulation, which feedback is referred to as input data. In the example of the visual prosthesis such feedback may be recorded by requesting the user to point towards a direction in which the user perceived the phosphene, or preferably, through a tactile device such as a touchscreen input device, in which the user indicates in which visual field location he or she perceived the phosphene.

Once the feedback is recorded as such input data, these are used in subsequent steps as anchor points, which illustrate absolute, measured instead of calculated, locations of the respective electrodes in absolute visual space.

The previously obtained relative location map comprising recorded data from all functioning electrodes, is combined with the input data, i.e. the anchor points, such that an absolute location map is obtained in which location data of each electrode is contained, and in which the positions of the electrodes relative to each other is known, but which positions are mapped to the absolute coordinates in visual space, through the anchor points.

As such, perceptual or functional location mapping is generated by which a visual prosthesis can be calibrated for a particular user, such that electrodes of the visual prosthesis can be driven in accordance with the visual perception location map, to substitute the visual modality of the user.

Similarly, as expressed, other functional modalities can be calibrated with the method according to the present disclosure as well. Therein the electrodes are implanted in respective brain regions corresponding to the functional modality which is to be substituted by the prosthesis. The driving and recording of the neural signals to generate maps of electrode locations relative to each other may be performed in a manner similar to that of the visual prosthesis explained above, where the steps of driving a subset of the electrodes and to obtain user input to perform manual mapping of the subset of electrodes to obtain absolute locations or anchor points may be performed in accordance with the respective functional modality, e.g. in case of auditory modality or motor prosthesis. The mathematical modelling of the sensory data is however similar and therefore the present disclosure is not limited to a visual prosthesis, but may also be embodied in several other neuroprosthetic systems which require a calibration of the implanted electrodes with the sensory perceptions or motor or other functions induced by the prosthesis.

In an example, at the step of obtaining input data, the input data is obtained from the mammal as feedback on driving a selected subset of electrodes via electrical stimulation of the region of the brain, and wherein the input data comprises a set of location data in absolute target space, and each of the location data corresponding to an electrode of the subset, and the selected subset comprises a limited amount of electrodes for manual mapping of the limited electrodes in the subset to the location data in the absolute target space.

The present disclosure is based on the insight of calibrating the intracortical prosthesis with both a highly automated mapping routine as well as a manual mapping routine. Whereas the automated mapping routine relies on transforming data elements sensed in an automated manner and converting each and every data element into a relative location map, the manual mapping routine relies on selecting a subset, preferably being a small number of electrodes. For the subset the electrodes are actually driven to stimulate whereas in the automated mapping routine the electrodes are only operated as sensors. For each electrode driven in the manual mapping routine input data is obtained through for example a feedback device such as a tactile feedback capable of obtaining user input corresponding to the functional modality that is to be replaced, e.g. device like a touchpad in case of a visual modality.

In an example, the method comprises, prior to the step of defining a relative location map, the additional step of:
- calculating a feature matrix representing patterns of implanted brain region locations, wherein said feature matrix comprises said neural dataset obtained from each electrode of the one or more electrode arrays, or wherein said feature matrix is calculated by determining a relation between the data of each electrode in the neural dataset, or a specific measure of the data of each individual electrode in the neural dataset, or a combination thereof, and wherein the step of defining the relative location map comprises the step of
- defining a relative location map from said calculated feature matrix.

Hence, in between the steps of obtaining the neural dataset and defining the relative location map, a feature matrix is calculated. The feature matrix may comprise all of the (raw) data from the neural dataset. These may in an example directly be transformed into the relative location map. However, in accordance with the example above, the neural dataset may also be pre-processed in such an intermediate step in which the data is filtered. The skilled person will appreciate which filtering or preprocessing may be suitable and as indicated in the example, may comprise a transformation defining a relation between data of each or a selection of electrodes in the neural dataset.

In an example, the method further comprising the step of storing the location data in the absolute target space as anchor points in the relative location map.

In an example, the region of the brain is the cerebral cortex of the brain, and in particular the primary visual cortex.

In addition to the cortex, the brain region could also include areas such as V2, V3 and V4, in addition to primary visual cortex (V1), or the LGN of thalamus, or any other brain region.

In an example, stimulation is electrical stimulation.

In an example, the input data is obtained through an eye tracker, a tactile sensor, in particular a pointing device, and more in particular a touchpad or trackpad.

In an example, the input data is obtained through a tactile sensor, in particular an eye tracker or a pointing device, and more in particular a touchpad or trackpad.

In an example, each of said electrode arrays comprises 16, 32, 64, 128 or 256, 512, 1024 or more electrodes.

Typically, the electrode arrays may comprise a relatively large number of electrodes, e.g. an array with 100+ electrodes, to cover the implanted brain region in such a sufficiently widespread manner that the sensory modality can be substituted in a functional manner.

In an example, the step of calculating location data for all electrodes, comprises geometric transformation of said location data for all electrodes.

In an example, the step of defining a relative location map from said calculated feature matrix comprises applying data-driven mapping algorithms for all electrodes, after which, an alignment method may be used such as Procrustes to calculate the final location data

In an example, the data-driven mapping algorithms comprises one or more of reinforcement learning, unsupervised, semi-supervised and supervised mapping algorithms.

Several algorithms may be used for the data-driven mapping, i.e. for the first automated mapping of all electrodes in each array in defining the relative location map. The algorithm may be selected based on a categorization of reinforcement learning, unsupervised, semi-supervised and supervised mapping algorithms. For an applicable example of unsupervised or semi-supervised algorithm dimensional reduction may be employed, for example in a linear or in a non-linear manner. For the linear manner, matrix decomposition, PCA (Principal Component Analysis), or ICA may be employed, whereas for non-linear, multidimensional scaling, t-SNE, UMAP or autoencoders may be employed. For supervised algorithms, also linear and non-linear categories may be used, in which linear regression would be an applicable example of linear, and supervised neural networks an applicable example of non-linear. The skilled person will however appreciate which other algorithms may also be applicable.

The electrodes may be classified into a group for automated mapping and a group of manual mapping, although, preferably, the group of electrodes for automated mapping comprises every implanted electrode, whereas the group of electrodes for manual mapping may comprise a small number of electrodes. This subset with manual mapping electrodes, preferably has absolute locations which are not located near to each other, but are equally or at least substantially well distributed over the region of the brain where the electrode arrays are implanted.

Before defining the relative location map before defining the absolute location map, additional steps may be introduced to enhance the models based on known neurological information. Although the anatomy of the brain of individuals may vary to a certain extent, overall anatomical information may be used to enhance the model. Known three-dimensional models of the cortex may be used in the relative and absolute map. Also, for those individual mammals for which the information is present, for example when the mammal has undergone brain imaging, the individual image data may be used alternatively or in addition to the general cortical models.

Moreover, the calibration routine presented, and in particular the relative and/or absolute maps may be further enhanced by examining neuronal activity of the mammal, for example when the eyes are closed and blindness is simulated. Also, known mapping techniques may be used for further enhancement, e.g. semi-automated methods for rough mapping of for example the boundaries of target space.

In a further aspect, there is provided a neuroprosthetic system, comprising a sensor, for use by a mammal using the system, arranged for generating a sensed data feed by sensing a neural modality of the brain of the mammal which is to be substituted, a plurality of electrode arrays, each comprised of a plurality of three-dimensional electrodes, arranged for intracortical implantation for a dense occupation of a region of the cerebral cortex of the mammal arranged for providing functional coverage of the sensory or motor or other modality, each electrode being arranged for electrical stimulation of subsets of locations in the region of the cerebral cortex, a driving unit, arranged for electrically driving the electrodes of the electrode arrays for stimulating the subsets of locations in the region of the cerebral cortex, a recording unit, arranged for obtaining neural recording through the electrodes in the region of the cerebral cortex, a processing unit, arranged for analysing the sensed data feed for providing stimulation patterns for electrically driving groups of electrical contacts of the electrode unit corresponding to subsets of locations in the region of the cerebral cortex, for substituting the sensory modality, and a memory comprising a phosphene location mapping for electrically driving the electrodes of the one or more electrode arrays for subsets of the region of the brain in accordance with the phosphene location mapping and wherein the phosphene location mapping is obtained by a computer-implemented method according to any of the previous aspects and/or examples.

In an aspect, the method may be used for, or implemented in an augmented neuroprosthetic system, e.g. arranged for augmented vision in which a brain-computer interface may provide additional information to a user by retrieving external information relevant for the current situation, e.g. to aid in recognizing faces or assist in traffic. The prosthesis and methods according to the present disclosure can thus be used for replacing functional modality, for example, for restoring of blindness or may also be used for supernormal functional modality such as supernormal sight which can be provided by augmented sensors.

In yet another aspect, there is provided a computer-implemented method and corresponding system for mapping transducers of a brain prosthesis for substituting a defective functional modality of a mammal with one or more transducer arrays for stimulation of and recording from a region of the brain of said mammal corresponding to a neural modality for which said functional modality is to be substituted, said method being performed by a computing unit and comprising the steps of:
- obtaining a neural dataset, wherein said neural dataset comprises data simultaneously sensed from each transducer of said one or more transducer arrays;
- defining a relative location map from said calculated neural dataset;
- obtaining input data, wherein said input data is obtained from said mammal as feedback on driving a selected subset of transducers for stimulation of said region of said brain, and wherein said input data comprises a set of location data in absolute target space, and each of said location data corresponds to an transducer of said subset;
- corresponding each of said location data with a data point in said relative location map, corresponding to said transducer of said subset;
- calculating location data for remaining transducers, not comprised in said subset;
- defining an absolute location map comprising location data of each transducer of said one or more transducer arrays, which map defines sensory perception locations in absolute visual space, and wherein said location data comprises both said calculated location data and location data obtained from said input data of said mammal;
- generating a sensory perception location mapping for electrically driving or recording from said transducers of said one or more transducer arrays for subsets of said region of the brain in accordance with said defined absolute distance map, for said functional modality.

In an example, the computer-implemented method comprises, prior to the step of defining a relative location map, the additional step of:
- calculating a feature matrix representing patterns of implanted brain region locations, wherein said feature matrix comprises said neural dataset obtained from each electrode of the one or more electrode arrays, or wherein said feature matrix is calculated by determining a relation between the data of each electrode in the neural dataset, or a specific measure of the data of each individual electrode in the neural dataset, or a combination thereof, and wherein the step of defining the relative location map comprises the step of
- defining a relative location map from said calculated feature matrix.

In the aspect described above, the method according to the present disclosure is used to map transducers instead of electrodes. Such transducers may be interpreted in a broad sense, e.g. chemical transducers or optrodes or any type of acoustic, visible or invisible light, electromagnetic, or gravitational radiation. Thus for example based on an array of ultrasound transducers, chemical or electrochemical actuators, magnets, etc.

The abovementioned and other aspects of the present disclosure will be apparent from and elucidated with reference to the embodiments described herein after.

### Brief Description of the Drawings

Fig. 1 illustrates, schematically, a brain prosthesis;
Fig. 2 illustrates a transducer unit and multiple three-dimensional arrays as part of a brain prosthesis;
Fig. 3 illustrates several steps of processing an image captured by the camera of a brain prosthesis;
Fig. 4 shows the steps of mapping electrodes of a brain prosthesis according to an aspect of the present disclosure;
Fig. 5a-d show details of the steps of mapping electrodes of a brain prosthesis according to an aspect of the present disclosure;
Fig. 6 and 7 show embodiments of the steps of mapping electrodes of a brain prosthesis according to an aspect of the present disclosure;

### Detailed description

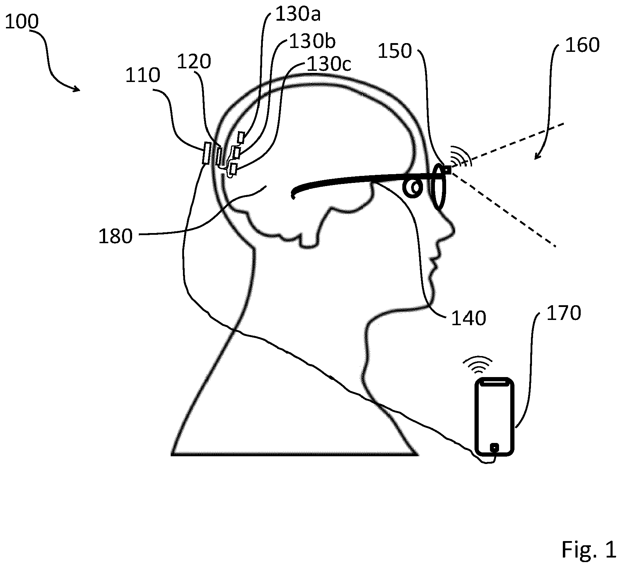

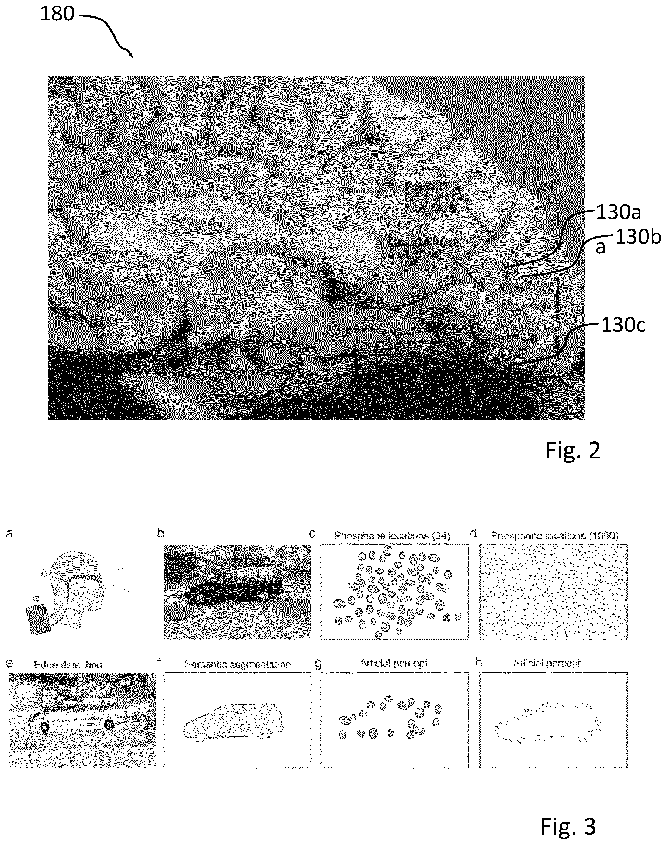

Fig. 1 and 2 schematically illustrate a brain prosthesis 100 and detail thereof for substituting a defective functional modality of a mammal with one or more electrode arrays for stimulation of and recording from a region of the brain of the mammal corresponding to a neural modality for which the functional modality is to be substituted.

Although the examples illustrated in the figures and described in the description below are directed to a visual neuroprosthetic system in which visual perception is substituted in the visual region of the cerebral cortex, the present invention is not limited solely to the visual sensory modality. The skilled person will appreciate that the present invention is also applicable for other functional modalities such as the auditory or motor or somatosensory modality. The skilled person will appreciate that several of the components described below may require replacing them with suitable and corresponding components. The sensor in a visual prosthetic system for example is typically an image sensor, whereas in the example of substituting a auditory modality, the sensor will be an audio sensor. The skilled person will appreciate where modifications may be required, which modifications are common knowledge, not relevant for the scope of the present disclosure, and as such, not further described throughout the description.

Merely as an illustration, the system 100 as demonstrated in Fig. 1, contains several separate physical units, which are contained in separate housings. The skilled person will appreciate that some units however, may also be combined into a single housing.

The system 100 consists of a sensor 150. The sensor may comprise a digital camera or cameras, photodetectors or photosensors, for operating in the visual and/or InfraRed, IR, spectrum, and mounted at glasses or the like. In the example shown in Fig. 1 the sensor is an image sensor which is mounted on glasses 140 of the patient. The sensor may however also be a separate camera that is attachable to the body or can be worn by patient in any other way. The camera may also be a separate hand-held camera unit or incorporated in a portable device such as a mobile phone or portable computer device like a tablet.

The camera 150 is pointed in a direction that corresponds to the field of view 160 of the patient. As such, the camera is able to record or capture a data stream, in this example a data stream consisting of a plurality of images. The data stream contains data or images that cover at least most of the field of view 160. The term field of view refers to the restriction of what is actually visible by the camera 150. Since the human eye may have a larger field of view than most camera devices, the system may also comprise an eye-tracking unit, which is not shown in Fig. 1. The eye-tracking unit is able to detect the position of the eye of the patient, and thus the visual field location to which the eye is directed. In this way, the camera device may be focused to his or her direction of gaze and generate a data stream that corresponds to the focus area in the field of view of the patient.

The system 100 also consists of an electrode unit 130. The electrode unit 130, in practice, consists of multiple three-dimensional arrays 130a, 130b, 130c. The system 100 consists preferably of approximately 20 of these arrays 130, for example 10 implanted into the left hemisphere and 10 in the right hemisphere.

Each three-dimensional array 130a, 130b, 130c consists of multiple electrodes or elongated electrode shafts. The electrode shafts are distributed in such a three-dimensional manner over the array that they are able to make electrical contact with a large three-dimensional region of the brain e.g. cerebral cortex, i.e. with most of, or approximately all of the visual region of the cerebral cortex of the patient. This means, that the spacing between the individual electrode shafts is small, e.g. between 0.4mm and 1 mm, and may have a length up to 20 or even 40 mm. Preferably however, the length of the electrode shafts are in the range of at least 2 to 5 mm till 20 or 40 mm. More preferably, the length is in the range of 5 till 30 mm, even more preferably in the range of 7 till 25 mm, and most preferably in the range of 15 till 20 mm. This way, the electrodes are able to extend beyond a single, or cover even plural folded gyri in the brain or in particular in the cerebral cortex.

Each electrode shaft may consist of multiple electrical contacts. These contacts may be controlled individually such that some of the contacts of a single electrode shaft are electrically driven or activated by applying a stimulation signal, and others are not operated. Each contact may also be operated for either signal stimulation or signal recording. In an operational mode, the contacts are operated for signal stimulation only. In a configuration mode, the contacts may however also be controlled to operate in a signal recording mode such that neural activity or neural response to the stimulation signal may be recorded accordingly. In an example, each contact may be arranged such that it can operate both in the stimulation and recording mode, and in an alternative example, contacts may be dedicated for either signal stimulation or signal recording.

In order for the electrical contacts to process the signal for neural stimulation or neural recording, the system 100 may consist of a driving unit and a recording unit. These units may be contained in separate housings, at separate locations in or on the patient, but are preferably contained in a transducer device which may be close to or integrated with the shaft or positioned directly under the scalp on the skull, for example in or near the layer of loose connective tissue of the scalp.

The electronics of the driving unit, to electrically drive the electrode unit for stimulation of the subset of locations, as well as the recording unit, to obtain neural recording through the electrode unit in the region of the cerebral cortex, can be packaged inside an implantable transducer casing 120 and shielded from the tissue by the material of the casing, which is preferably made of titanium. In case the electronics or electronic circuitry may reside on the electrode unit or more particular, on the three-dimensional arrays, they are embedded in a tissue-compatible packaging material such as LCP or polyimide.

The implant 120 may also consist of a switching unit. The switching unit consists of electronic circuitry which channels stimulation signals from the driving unit to the actual electrical contacts located in the cerebral cortex or other parts of the brain, depending on the type of sensory functionality. What that means is, that the driving unit may be equipped with circuitry to generate a plural stimulation signal, i.e. a stimulation pulse of a particular waveform such as a sine wave form, a square wave form, a rectangular wave form, a triangular wave form, a sawtooth wave form, a pulse wave form or any combination of wave forms. The generated signals may not only differ in shape but also in amplitude and can be channelled to different individual or subsets of electrical contacts. This way some groups or subsets of contacts may not be activated at all, others with a first stimulation signal, yet another group with a second, different stimulation signal, and so on. To this end, the switching unit is arranged to achieve electrical channelling of the different signal generators and the subsets of electrical contacts.

To control the driving unit, the recording unit, and thereby the electrodes of the electrode unit, the system may be provided with a processing unit 170. The processing unit is preferably a handheld device, since that will increase independence and quality of life of the patient. The processing unit or device could be a general-purpose hand-held device, such as the mobile phone as shown by way of example in Fig. 1, which is programmed to operate as processing unit 170 for the brain system 100 according to the invention. The processing unit or device is however more preferably a dedicated device but with dimensions that are preferably similar to those of a mobile phone.

The processing unit 170 may communicate with the transducer arrangement or transducer device 120 by use of a wireless communication unit 110. This wireless communication unit 110 not only provides wireless technology to control high-channel-count high-density cortical implantable arrays 130a-130c, but also provides a wireless power transfer interface that can send the signals of the recording unit. This can be achieved for example through capacitive, or magnetodynamic, but preferably through inductive coupling between the wireless unit 110 and the transducer implant 120.

By having both wireless power and data communication between the wireless unit 110 and the transducer implant 120, the risk of infection that is associated with skin-penetrating cables and connectors is reduced.

With the processing unit 170, the system 100 is able to receive and analyse the data stream sensed by the sensor. In the example shown in Fig. 1, these are images of the data stream captured by the camera 150. These images have to be processed in such a way that the correct electrical contacts are powered or provided with a stimulation signal to evoke a phosphene, i.e. a phenomenon characterized by the experience of seeing light or a specific light dot, at a phosphene location which corresponds to the actual position of the electrode shaft or more precisely, the electrical contact of that particular electrode shaft. The subset of phosphene locations should correspond to one or more objects that are captured by the camera and thus in the scene in the field of view 160 of the patient.

Before being able to make such a translation between actual captured image data and the subset of phosphene locations (which together form a phosphene pattern or patterns), the processing unit 170 needs to be calibrated or configured.

Implanting several electrode arrays will provide numerous locations in a respective region of the brain or cerebral cortex providing functional coverage of the sensory modality.

However, as it may be unclear what the location of the electrodes in the brain structure is, a mapping of the electrodes of the prosthesis is required before it can be used. Calibrating or configuring the prosthesis prior to use is therefore required and is based on determining the relation between the channel or electrode identity on the prosthesis, and the location of the electrodes in the brain structure/functional map. From the location of electrodes within the map, the location of phosphenes can deduced, i.e. in case of a visual prosthesis.

Once the system is calibrated or configured, the processing unit 170 can process the image/video data, and output an outline, contour, label, shape or other primitive object or image spatiotemporal feature which can then be used to apply stimulation signals to the appropriate subset of phosphene location. Once these stimulation signals are applied, the invoked phosphenes at these phosphene locations will substitute the visual perception of the patient such that he or she will recognize the actual object as a car or whatever object is in the field of view 160 of the patient. These steps a. throughout h. are shown in Fig. 3.

To date, known visual neuroprosthesis systems in blind human subjects have employed manual mapping techniques to determine this location, of which there are several variants (described below).
- Stimulation is delivered to one electrode at a time, and the implant user reports the location of the perceived phosphene in absolute coordinates of visual space, e.g. by placing their finger on an electronic touch pad or a tactile surface; or by indicating the location using a wand that is coupled to a visual tracking system.
- Stimulation is delivered to two electrodes successively, and the implant user reports the relative positions of the perceived phosphenes, e.g. by reporting the radial angle between the first and second phosphenes.

The two variants of manual phosphene mapping described above can be combined, to provide a more refined map. For example, the first approach could be carried out to obtain a gross map of phosphene locations across electrodes, followed by the use of the second approach to fine-tune the relative locations of phosphenes that are close to each other in the gross map.

Regardless of the variant that is used (or combination thereof), manual mapping has several drawbacks:
1) Manual reporting is a time-consuming process, which needs to be carried out for each electrode or combination of electrodes, and requires the user to remain alert and perform the task as accurately as possible. Accurate and systematic phosphene mapping, when done manually, can take days to weeks, if successful.
2) Some blind individuals experience a phenomenon known as 'spontaneous phosphenes,' in which they perceive spontaneously occurring flashes or dots of light (also known as 'phosphenes') that are entirely decoupled from their visual surroundings, making it difficult for subjects to detect and locate electrically induced phosphenes. It may take weeks or months of training and rehabilitation before subjects can reliably identify and distinguish artificially induced phosphenes from naturally occurring ones, significantly delaying the use of artificial vision.
3) Many blind individuals, including those with acquired blindness, exhibit abnormal eye movements, particularly if they have been blind for an extended period of time. This includes making spontaneous eye movements, overshooting of saccade targets, and nystagmus (the tendency for the eyes to drift away from a target). These abnormalities are thought to arise due to chronic visual deprivation and the lack of feedback to the ocular muscles following the onset of blindness. In normally sighted individuals, lights impinges on the retina before being sent to the brain via the optic nerve, and the brain takes the eye position into account when interpreting the incoming images. By contrast, in a visual cortical prosthesis system, light is captured by a camera and electrical stimulation is delivered directly to the brain, bypassing the delivery of light signals to the eye. However, the brain continues to compensate for the user's eye position and direction of gaze, hence phosphenes move whenever the eye moves. The presence of disordered eye movements can make it challenging for subjects to accurately report the locations of phosphenes during the manual mapping process.

In summary, known techniques are not readily scalable to large numbers of electrodes; they are inefficient in terms of time and energy spent in the clinic; and they may not work for certain groups of blind users (e.g. those with spontaneous phosphenes or abnormal eye movements).

The present disclosure is based on the insight of a semi-automated approach to phosphene mapping, which is scalable, efficient, and remains robust even in the presence of spontaneous phosphenes and abnormal eye movements. Which approach is defined in several aspects of the present disclosure and also applicable and suitable for other brain prostheses in which a defective functional modality of a mammal is substituted with one or more electrode arrays.

What is proposed is that neuronal signals are recorded simultaneously from the implanted electrodes in the absence of visual input, hence, a neural data set is obtained. This can be carried out whether the user is awake or asleep. Using the presented method, maps are extracted of correlated activity between channels, hence, a feature relative location map may be defined, which acts as a proxy for the underlying anatomical and functional connectivity between neurons across channels. Thus far, the steps involved have not required any manual mapping on the part of the user, and can be carried out across a large number of electrodes.

Next, manual mapping is carried out using traditional techniques, but only for a small, manageable subset of electrodes. Thus, input data is obtained wherein feedback of the user for the subset of electrodes is obtained which yields a set of 'anchor points' consisting of accurate coordinates of phosphene locations in absolute visual space, for this subset of channels. These manually obtained anchor points are combined with the automatically derived maps of correlated neuronal activity, by corresponding and calculating the location data for the electrodes using certain algorithms, yielding accurate, high-channel-count maps of phosphene locations in visual space which are stored as a sensory perception location mapping which can be used to calibrate a brain prosthesis for a particular user.

With the use of high-throughput, fully automated data recording and processing techniques to handle the bulk of the data, combined with a rapid and low-channel-count manual mapping process, accurate, large-scale maps of phosphene locations in a blind user are generated.

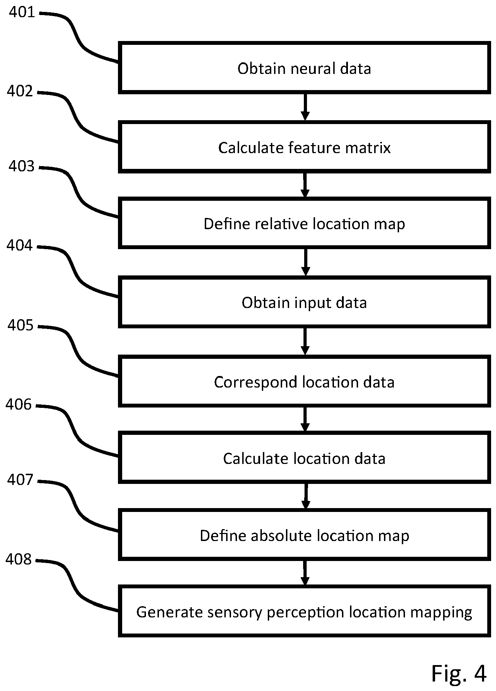

This concept is defined and shown in Fig. 4 which shows the flowchart of the computer-implemented method according to an aspect of the present disclosure, for mapping electrodes of a brain prosthesis for substituting a defective functional modality of a mammal with one or more electrode arrays for stimulation of and recording from a region of the brain of the mammal corresponding to a neural modality for which the functional modality is to be substituted, the method being performed by a computing unit.

The method comprises several steps, which preferably, but not necessary may be performed in sequential order.

In the first step, 401 a neural data set is obtained. This neural data set is also referred to as "neural data matrix". The dataset comprises data simultaneously sensed from each electrode of the one or more electrode arrays.

In the next, second step, 402, a feature matrix is calculated, which represents a pattern of implanted brain region locations, wherein the feature matrix comprises the neural dataset obtained from each electrode of the one or more electrode arrays, or wherein the feature matrix is calculated by determining a relation between the data of each electrode in the neural dataset.

The feature matrix may be a correlation matrix or any other type of matrix defining a relation between data of the neuronal dataset. The relation may in particular cover any of correlation, variance, covariance, level of similarity, spectral features, fourier coefficients, or entropy measures. In addition, the feature matrix might contain exclusively features corresponding to each neural data channel, or a mix between features corresponding to each neural data channel and features representing relationships between the said channel and the rest of the channels.

Next, in step 403, a relative location map is defined from the calculated feature matrix.

Then, input data 404 is obtained, wherein the input data is obtained from the mammal as feedback on driving a selected subset of electrodes for stimulation of the region of the brain, and wherein the input data comprises a set of location data in absolute target space, and each of the location data corresponds to an electrode of the subset.

In the next step 405, each of the location data is corresponded with a data point in the relative location map, corresponding to the electrode of the subset.

Then, location data is calculated 406 for all electrodes.

In step 407 an absolute location map is defined comprising location data of each electrode of the one or more electrode arrays, which map defines sensory perception locations in absolute visual space, and wherein the location data comprises both the calculated location data and location data obtained from the input data of the mammal.

Finally, in step 408 a sensory perception location mapping is generated for electrically driving or recording from the electrodes of the one or more electrode arrays for subsets of the region of the brain in accordance with the defined absolute location map, for the functional modality.

The present disclosure is based on the fact that one may consider two separate reference frames for the maps of the present disclosure, which maps for the visual prosthesis can be in either retinal or cortical coordinates. 'Retinal coordinates' are equivalent to visual field coordinates, i.e. within the visual field, and are specified in units of degrees of visual angle (dva). 'Cortical coordinates' are the locations in the cortex of the brain and are specified in distance such as millimetres. When traditional phosphene mapping is carried out, patients give the locations of phosphenes in retinal coordinates. Similarly, for the phosphene locations obtained in step 404, using the input data from a subset of electrodes, the coordinates are in the retinal frame of reference. Specific methods may be employed to convert coordinates between cortical and retinal reference frames. The present disclosure provides a novel method to primarily generate maps within a cortical reference frame, at least when the electrodes are located in a single visual area, hence the cortical coordinates are converted into retinal coordinates (or vice versa) using selected methods.

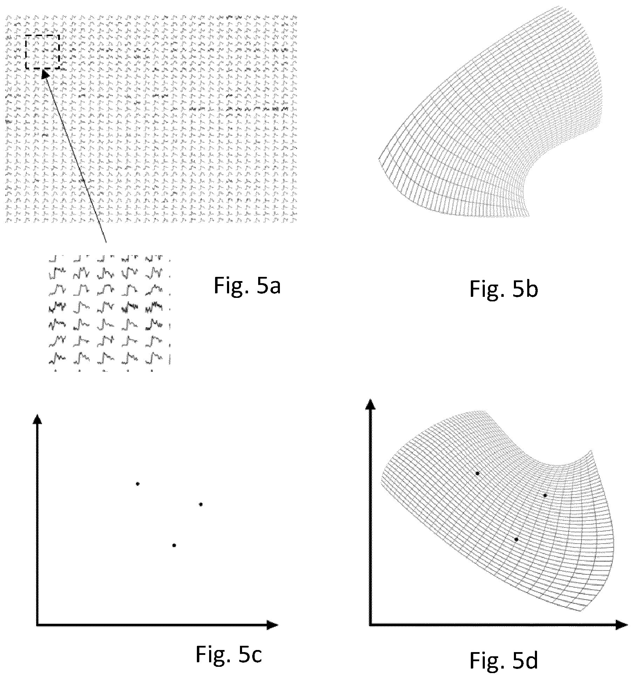

In Fig. 5a to 5d the method above is illustrated in a simplified manner to visualize how the mapping is performed.

In the first step, shown in Fig. 5a, neuronal signals are recorded as a neural dataset, from many electrodes in a simultaneous manner. The figure also shows a detail in which each individual recording is shown, for 30 electrodes. The neuronal signals are obtained from each electrode of each array for a duration of a certain pre-defined time-period, generating a dataset of *n-by-t.* The signals may subsequently be filtered along dimension *t* to generate filtered signal, and dimensionality reduction may be performed to filtered signal to reduce its dimension from *n-by-t* to *n-by-2* or *n-by-3.*

In the second step, shown in Fig. 5b, algorithms are used to process the recorded data elements of the neural dataset or the dimension-reduced dataset, and to generate a map of relative locations or in the present example of the visual prosthesis, a relative electrode location map, i.e. the locations of all of the electrodes, where the locations are relative to each other. Each vertex in the figure represents the relative location of an electrode.

In the third step, shown in Fig. 5c, for a limited number of electrodes, a manual mapping is carried out, while the user provides feedback in the form of phosphene locations. Optionally, the mentioned phosphene locations can be projected into cortical coordinates. This provides several anchor points, either within the visual space, or within the cortical space.

In the final step, shown in Fig. 5d, the coordinates of the anchor points and the relative maps that were derived from the recorded data in step 2, are combined, to yield an absolute location map, comprising either absolute cortical coordinates that can be later projected to absolute coordinates of visual space, or absolute phosphene locations in which not only the positions of the phosphenes relative to each other are defined, but also in absolute coordinates in visual space. The final step shown in Fig. 5d may comprise two sub-steps: the data are combined to yield an absolute location map of the electrodes in cortical coordinates, and then this is converted (e.g. using existing methods) into retinal coordinates; the retinal coordinates are the phosphene locations in absolute visual space

Combining the relative data and anchor points into the absolute map may comprise scaling, warping and/or rotation of the relative map as visualized in the Figures 5a-d. Further, in another embodiment, evaluating the actual effects of the neuro-intervention may also performed against its intended effect by means of subjective measurement or user feedback. And in another embodiment, the parameters of the dimensionality reduction algorithm may be adjusted based on the evaluation to produce an updated relative map. Also, in yet another embodiment, the locations of s electrodes may be used in the absolute map to transform the updated relative map, e.g. by scaling and/or rotation. In yet another embodiment, the procedural steps may be repeated until an optimal map is found judging by evaluating the actual effects of neuro-intervention performed by the chosen subset of electrodes against its intended effects.

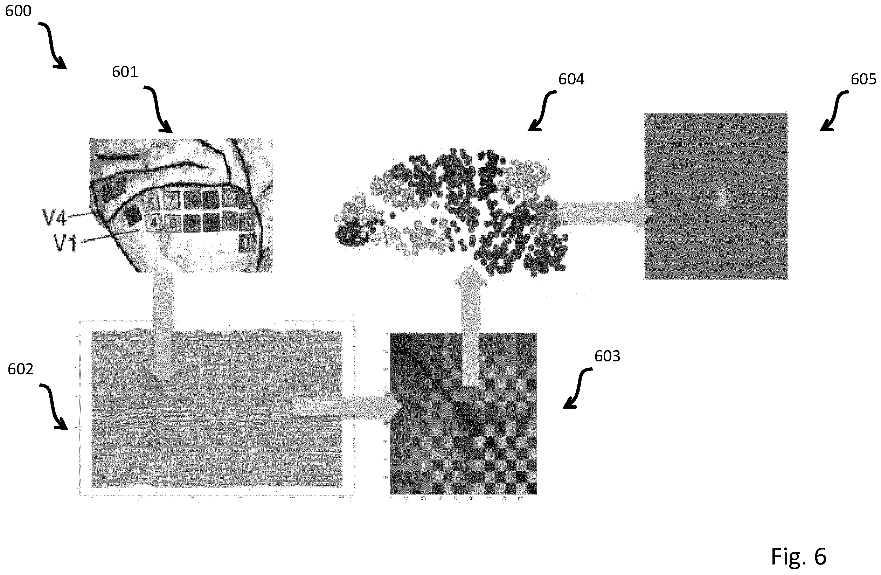

In Fig. 6 an embodiment is shown of the present disclosure in which through steps 601 to 605 a sensory perception location mapping may be obtained. In the first step, 601, sixteen electrode arrays are implanted in several regions of the brain. As illustrated in Fig. 6, most of the arrays are, by way of example, implanted in the primary visual cortex, and some in the visual area V4. From each of the electrodes a neuronal signal may be recorded for a set period of time. These recordings 602 are converted into a feature matrix 603, in this example a distance matrix comprising cross-correlations. The matrix is mapped into a relative electrode mapping 604 and into a phosphene mapping 605 which is used as the sensory perception location mapping to calibrate the visual prosthesis.

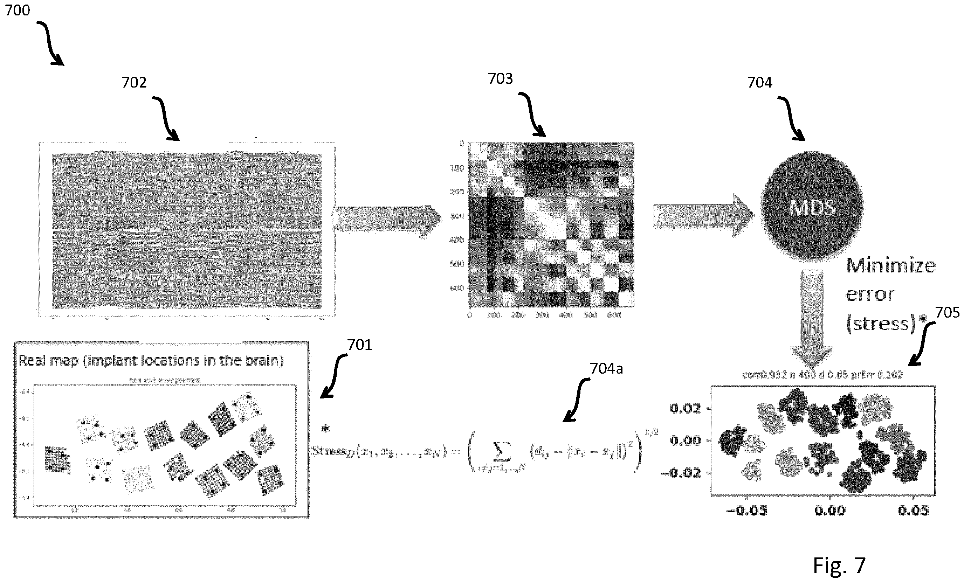

In Fig. 7 another embodiment is shown of the present disclosure in which through several steps a sensory perception location mapping 705 may be obtained. In the first step, 702, a similar to step 602 of Fig. 6, a neuronal signal may be recorded for a set period of time from each of the electrodes as implanted according to map 701. These recordings 702 are converted into a feature matrix 703, in this example a similarity matrix. The matrix is fed to an MDS algorithm 704 to obtain map 705 of relative electrode locations and thereby absolute phosphene locations by performing an error minimization (stress) routine as defined by 704a. The electrode mapping 705 and phosphene mapping can be used as the sensory perception location mapping to calibrate the visual prosthesis. The feature matrix or similarity matrix or distance matrix 703 comprises distances between each pair of objects in the neural dataset. The MDS algorithm places, for a chosen number of dimensions, *N,* each object into N-dimensional space (a lower-dimensional representation) such that the between-object distances are preserved as well as possible. It takes an input matrix giving dissimilarities between pairs of items and outputs a coordinate matrix whose configuration minimizes a loss function called stress, which may be defined by 704a.

Other variations to the disclosed examples can be understood and effected by those skilled in the art in practicing the claimed disclosure, from a study of the drawings, the disclosure and the appended claims. In the claims, the word "comprising" does not exclude other elements or steps and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as optical storage medium or a solid-state medium supplied together with or as part of other hardware, but may also be distributed in other forms, such as via the internet or other wired or wireless telecommunication systems. Any reference signs in the claims should not construed as limiting scope thereof. Similar reference signs denote similar or equivalent functionality.

The present disclosure is not limited to the examples as disclosed above, and can be modified and enhanced by those skilled in the art beyond the scope of the present disclosure as disclosed in the appended claims without having to apply inventive skills and for use in any data communication, data exchange and data processing environment, for example for use of the neuroprosthetic system for substituting auditory perception or motor control.

## Claims

1. A computer-implemented method for mapping electrodes of a brain prosthesis for substituting a defective functional modality of a mammal with one or more electrode arrays for stimulation of and recording from a region of the brain of said mammal corresponding to a neural modality for which said functional modality is to be substituted, said method being performed by a computing unit and comprising the steps of:
- obtaining a neural dataset, wherein said neural dataset comprises data simultaneously sensed from each electrode of said one or more electrode arrays;
- defining a relative location map from said neural dataset;
- obtaining input data, wherein said input data is obtained from said mammal as feedback on driving a selected subset of electrodes for stimulation of said region of said brain, and wherein said input data comprises a set of location data in absolute target space, and each of said location data corresponding to an electrode of said subset;
- corresponding each of said location data with a data point in said relative location map, corresponding to said electrode of said subset;
- calculating location data for remaining electrodes, not comprised in said subset;
- defining an absolute location map comprising location data of each electrode of said one or more electrode arrays, which map defines sensory perception locations in absolute visual space, and wherein said location data comprises both said calculated location data and location data obtained from said input data of said mammal;
- generating a sensory perception location mapping for electrically driving or recording from said electrodes of said one or more electrode arrays for subsets of said region of the brain in accordance with said defined absolute distance map, for said functional modality.

2. The computer-implemented method for mapping the electrodes of a brain prosthesis according to claim 1, wherein the method comprises, prior to the step of defining a relative location map, the additional step of:
- calculating a feature matrix representing patterns of implanted brain region locations, wherein said feature matrix comprises said neural dataset obtained from each electrode of the one or more electrode arrays, or wherein said feature matrix is calculated by determining a relation between the data of each electrode in the neural dataset, or a specific measure of the data of each individual electrode in the neural dataset, or a combination thereof, and wherein the step of defining the relative location map comprises the step of
- defining a relative location map from said calculated feature matrix.

3. The computer-implemented method for mapping the electrodes of a brain prosthesis according to claim 1 or 2, wherein at said step of obtaining input data said input data is obtained from said mammal as feedback on driving a selected subset of electrodes via electrical stimulation of said region of said brain, and wherein said input data comprises a set of location data in absolute target space, and each of said location data corresponds to an electrode of said subset, and said selected subset comprises a limited amount of electrodes for manual mapping of said limited electrodes in said subset to said location data in said absolute target space.

4. The computer-implemented method of mapping electrodes of a brain prosthesis according to claim 3, said method further comprising the step of storing said location data in said absolute target space as anchor points in said relative location map.

5. The computer-implemented method of mapping electrodes of a brain prosthesis according to any of the previous claims, wherein said region of the brain is the cerebral cortex of said brain, and in particular the primary visual cortex.

6. The computer-implemented method of mapping electrodes of a brain prosthesis according to any of the previous claims, wherein said stimulation is electrical stimulation.

7. The computer-implemented method of mapping electrodes of a brain prosthesis according to any of the previous claims, wherein said input data is obtained through a tactile sensor, in particular an eye tracker or a pointing device, and more in particular a touchpad or trackpad.

8. The computer-implemented method of mapping electrodes of a brain prosthesis according to any of the previous claims, wherein each of said electrode arrays comprises 16, 32, 64, 128 or 256, 512, 1024 or more electrodes.

9. The computer-implemented method of mapping electrodes of a brain prosthesis according to any of the previous claims, wherein said step of calculating location data for all electrodes, comprises geometric transformation of said location data for all electrodes.

10. The computer-implemented method of mapping electrodes of a brain prosthesis according to any of the previous claims, wherein said step of calculating location data for all electrodes comprises applying data-driven mapping algorithms for all electrodes.

11. The computer-implemented method of mapping electrodes of a brain prosthesis according to claim 10, wherein said data-driven mapping algorithms comprises one or more of reinforcement learning, unsupervised, semi-supervised and supervised mapping algorithms.

12. A brain prosthetic system for substituting a defective functional modality of a mammal with one or more electrode arrays, comprising a sensor, for use by a mammal using said system, arranged for generating a sensed data feed by sensing a neural modality of the brain of said mammal which is to be substituted, a plurality of electrode arrays, each comprised of a plurality of three-dimensional electrodes, arranged for implantation in a region of the brain corresponding to the neural modality for which said functional modality is to be substituted, wherein said electrode arrays provide a widespread occupation of a region of the brain, each electrode being arranged for electrical stimulation of subsets of locations in said region of the brain, a driving unit, arranged for electrically driving said electrodes of said electrode arrays for stimulating said subsets of locations in said region of the brain, a recording unit, arranged obtaining a neural dataset through neural recording of said implanted electrodes, a processing unit, arranged for analysing said sensed data feed for providing stimulation patterns for electrically driving groups of electrical contacts of said electrode unit corresponding to subsets of locations in said region of the brain, for substituting said defective functional modality, and a memory comprising a sensory perception or functional location mapping for electrically driving said electrodes of said one or more electrode arrays for subsets of said region of the brain in accordance with said sensory perception or functional location mapping and wherein said sensory perception or functional location mapping is obtained by a computer-implemented method according to any of the previous claims 1-11.
